# EUROPEAN PATENT APPLICATION

(11) **EP 1 600 500 A1**
(43) Date of publication of application: **30.11.2005**
(21) Application number: 04012622.9
(22) Date of filing: 27.05.2004
(51) Int. Cl.: C12N 5/06

(54) **Method for producing pluripotent cells from monocytes**

(71) Applicant: Georg-August-Universität Göttingen, 37099 Göttingen (DE)
(72) Inventor: Peters, Hinrich J., 37077 Göttingen (DE); Heinemann, Dagmar, 37077 Göttingen (DE)
(74) Representative: Schüssler, Andrea

(57) **Abstract**

The present invention relates to methods for producing a cell population containing a majority of pluripotent intermediate cells or the pluripotent intermediate cells as such from monocytes, the pluripotent intermediate cells obtainable by said methods and their use for research purposes, diagnostic purposes, therapeutic purposes and/or further differentiation.

## Description

The present invention relates to methods for producing a cell population containing a majority of pluripotent intermediate cells or the pluripotent intermediate cells as such from monocytes, the pluripotent intermediate cells obtainable by said methods and their use for research purposes, diagnostic purposes, therapeutic purposes and/ or further differentiation.

Stem cells are cells found in all vertebrate animals, including human beings. They play a dominant role in the processes of normal development and regeneration or repair of damaged tissues. The reason for this is their capability of dividing to give rise to cells either identical to themselves or differentiated into particular types of cells. Cells having such developmental plasticity, i.e. cells not fixed as to their developmental destiny, are also referred to as pluripotent cells.

Because of their unique properties, pluripotent stem cells may find use in research, diagnosis, and therapy. Potential uses for pluripotent stem cells or their mature progeny include their application for studying fundamental processes of human development, such as differentiation, for toxicological testing and drug design, and for transplantation to replace cells or tissues damaged, e.g. by disease. However, the use of stem cells of fetal and/ or embryonic origin raises a number of ethical and legal questions. It would therefore be advantageous to use somatic stem cells, preferably from a readily accessible adult tissue.

Previous studies have shown that a number of adult somatic stem cells having a plasticity much higher than previously envisaged can be found in vertebrates. One example for somatic stem cells are monocytes. The pluripotent nature of monocytes has been documented by their ability to be transdifferentiated in numerous types of differentiated cells, either of hematopoietic or non-hematopoietic lineages.

Monocytes are well-defined white blood cells originating from bone marrow. Upon certain stimuli, they migrate into the tissues and further develop into macrophages, myeloid dendritic cells, osteoclasts, or microglial cells. As amoeboid or phagocytic cells, they belong to the phylogenetically very ancient cells that can be found in primitive organisms lacking lymphocytes or other elements of a specific immune system. Monocytes thus represent immune cells as well as components of inflammation. Recently, information accumulated that monocytes also participate in regeneration.

As to the developmental potential of monocytes, it was first discovered that monocytes are the precursors of macrophages. It was further demonstrated that monocytes have the potential of developing into myeloid dendritic cells (Peters et al., 1987). Monocytes were shown to represent the only nucleated cells in the blood failing to express nuclear lamine of the A/C variant, thus showing characteristics of precursor cells rather than of fully differentiated cells (Rober et al., 1990).

Following the discovery that myeloid dendritic cells derive from the monocytic lineage, it has been found that fibroblasts, microglial cells and osteoclasts also derive from the myeloid lineage. More recently, particular subsets of monocytes have been acknowledged as stem cells. Consequently, hepatocytes, adipocytes (Kuwana et al., 2003), myocytes (Kuwana et al., 2003), and nerve cells (Zhao et al., 2003) have been claimed to be transdifferentiated from monocytes.

However, the pathway of this transdifferentiation could not adequately be described in previous publications in as much as either the starting cell population, or the cell culture conditions, or the inducers were ill defined.

(Huss et al., 2000) isolated peripheral mononuclear cells from dogs, cultured them with IL-6 and obtained fibroblastoid cells which could be immortalized after 10-14 days. These cells transcribed the stem cell marker CD34 but did not express it. The cells expressed osteocalcin as a mesenchymal marker. They eventually differentiated spontaneously into CD34+ cells which showed colony formation. They could be used for autologous stem cell transplantation and reconstituted the blood of the dogs, demonstrating their pluripotent nature.

(Abe et al., 2001) started with CD14+ mononuclear cells and produced fibrocytes. The authors claim that cell contact with lymphocytes induces this process (without defining the signals), which could further be increased by the use of TGFbeta1.

(Yang et al., 2002) describe blood-born fibrocytes which belong to the adherent fraction of blood mononuclear cells and express type I collagen. Fibrocytes could be produced from CD14+ cells (not referred to as monocytes here). As an inducer, conditioned medium from CD14- cells was used. When adding TGFbeta, the formation of collagen+ cells was enhanced. Anti-TGFbeta obliterated the effect, so that TGFbeta is regarded as a main inducer of fibrocyte development. In burn patients these cells occur at higher frequencies in the peripheral blood than in healthy donors, correlating with a higher level of TGFbeta in the blood.

(Fernandez Pujol et al., 2000) used human monocytes to which angiogenic growth factor was added. After one week, endothelial cells expressing von Willebrand factor, CD144 (VE-cadherin), CD105 (endoglin), acetylated low-density lipoprotein (AC-LDL)-receptor, CD36 (thrombospondin receptor), FLT-1 (vascular endothelial growth factor receptor-1, VEGF-receptor-1) and KDR (VEGF-receptor-2) were obtained. In addition to these endothelial markers the cells expressed markers of the macrophage family, namely CD68, CD80, CD86, HLA-DR and CD36. The cells failed to express dendritic-cell markers such as CD1a and CD83.

(Zhao et al., 2003) used a subpopulation of monocytes, which required the addition of 10% fetal bovine serum to the culture. The authors induced differentiation by adding M-CSF. LIF or IL-6 enhanced the effect of M-CSF. After 14d of culture, cells termed "fibroblastoid macrophages" being positive for MAC-1 (CD11b/CD18), CD14, CD34, and CD45 were obtained. Diminished expression was found for HLA-DR, HLA-DQ, TNFRII, TNFalpha, leptin, PPARgammall, and lipid droplets.

(Abuljadayel, 2003) started with undefined cells isolated from peripheral blood mononuclear cells. The initial culture medium contained 10% fetal calf serum plus 10% horse serum, 10⁻⁷ M cortisol, and the monoclonal CR3/43 antibody as inducers for transdifferentiation. The CR3/43 antibody is directed against monomorphic regions of the human major histocompatibility complex (MHC)-class II antigens DP, DQ, and DR. Cortisol could be replaced by citrated human autologous plasma. Starting within one hour of culture and typically after 24 hours of culture, the cells showed the following marker combination: CD34+, CD19-, CD45low, CD38+ or -, CD133 (c-Kit)+ or -. Under clonogenic test conditions, these cells formed colonies of all hematopoietic lineages, demonstrating their pluripotent nature.

(Kuwana et al., 2003) obtained cells termed "monocyte-derived mesenchymal progenitors" (MOMP) from a subset of monocytes and further differentiated them into various distinct mesenchymal lineages. To obtain MOMPs, fibronectin coated surface, fetal bovine serum and conditioned medium from peripheral blood CD14- cells were used in the culture as inducers. The cells were shown to be proliferative over five passages. They could further be differentiated into mesenchymal cells such as osteoblasts, myoblasts, and adipocytes.

The cited studies suffer from the fact that the biological signals mediating the differentiation process are not yet fully understood. In these studies, *in vitro* differentiation has often been achieved by stimulation with unphysiological stimuli such as phorbol myristate acetate (TPA). Moreover, in the state of art neither the starting population of the cells nor the culture conditions are fully defined. Therefore, the route of transdifferentiation remained unclear so far.

In general, studies on the developmental potential of monocytes and the signals required for their differentiation can only be performed *in vitro,* provided the conditions are defined. In the previous studies cited above, monocytes were transdifferentiated into other cells either only from subpopulations of monocytes or under undefined culture conditions (i.e. in the presence of sera, conditioning factors, feeder cells), or both. However, a method enabling a universal way of transdifferentiating monocytes has not been described so far.

The technical problem underlying the present invention is the provision of a method for producing cells containing a majority of pluripotent somatic cells or the pluripotent somatic cell as such.

According to the present invention, this problem is solved by a method for producing a cell population containing a majority of pluripotent intermediate cells comprising the steps of
a. culturing a highly purified preparation of monocytes in the presence of at least one retrodifferentiation inducing agent and optionally GM-CSF, until a majority of the monocytes has attained the state of an intermediate cell; and
b. recovering the cell population containing a majority of pluripotent intermediate cells.

Alternatively, the problem is solved by a method for producing a pluripotent intermediate cell comprising the steps of
a. culturing a highly purified preparation of monocytes in the presence of at least one retrodifferentiation inducing agent and optionally GM-CSF; and
b. recovering a pluripotent intermediate cell.

Using the methods of the present invention, monocytes may be reproducibly converted into an intermediate stage of pluripotency (pluripotent intermediate cells) under defined conditions and in a quantitative manner. That is, not only a subpopulation of monocytes but the majority of monocytes or even at least 80% of the monocytes may attain the state of an intermediate cell using the methods of the present invention. The process of retrodifferentiation may thus be induced in more of 50%, preferable in more than 60%, and even more preferably in more than 70%, 80% or even 90% of the monocytes.

The terms "pluripotent intermediate cell", "intermediate cell" and "pluripotent cell" are used interchangeably for the purpose of this invention and refer to somatic stem cells having a distinct phenotype characterized by a marker combination shown in Table 1 below:

**Table 1:**

| Typical phenotypic markers for monocyte-derived pluripotent intermediate cells (determined for cells typically harvested after 10-15 days of culture) | |
|---|---|
| • HLA-class I | positive or diminished |
| • HLA-class II | positive or diminished |
| • CD 14 | diminished |
| • CD68 | positive or diminished |
| • Alkaline phosphatase (AP) | positive* |
| • CD35 variably | positive |
| • IL1beta | negative |
| • TNFalpha | negative |
| • CD54 | negative |
| • CD106 | negative |

| | |
|---|---|
| *) the AP+ state may be transient | |

The pluripotent intermediate cell of the invention thus phenotypically differs from all cell stages described in the state of the art up to date.

The term "highly purified" as used herein refers to a cell preparation containing at least 90% of monocytes. The term "monocyte" refers to cells of the monocyte-macrophage lineage, including mature monocytes, their descendants, and/or their precursors, carrying at least one marker of the myeloid lineage, i.e. CD14, CD68 (specific for mature cells) and/or CD33 (specific for their myeloid precursors).

The present authors found that monocytes may quantitatively be induced to transdifferentiate into other cell types under certain, well-defined cell culture conditions. For the purpose of this invention, the term "transdifferentiation" refers to the transformation of a differentiated cell of a given cell type to a differentiated cell of another cell type. The first step in said transdifferentiation process leads to a less differentiated, pluripotent intermediate cell and is achieved by the method described above. This first step is also referred to as "retrodifferentiation" for the purpose of this invention. Retrodifferentiation may be followed by a second step, i.e. differentiation into any other cells accessible from the intermediate pluripotent cell.

In detail, retrodifferentiation of monocytes to produce pluripotent intermediate cells is achieved by culturing the monocytes in the presence of at least one retrodifferentiation inducing agent and optionally GM-CSF. The term "at least one" means that up to 100 retrodifferentiation inducing agents may be used. Preferably, one, two or three, ten, 25 or 50 retrodifferentiation inducing agent(s) are combined.

In the context of the present invention, the term "retrodifferentiation inducing agent" refers to an agent that induces retrodifferentiation in monocytes resulting in the pluripotent intermediate cells according to the invention. For instance, the body's innate transmitters related to immunosuppression, such as corticosteroids (e.g. cortisol, hydrocortisone), interleukin 10 (IL-10), transforming growth factor β (TGFβ), interferone β (IFNβ), and vascular endothelial growth factor (VEGF), as well as all factors that counteract the action of typical immune activators, may be used to induce retrodifferentiation. Examples for immune activators are interleukin 1 (IL-1), interleukin 2 (IL-2), interleukin 12 (IL-12), interferon γ (INFγ), interleukin 8 (IL-8), IFNγ -inducing factor, tumor necrosis factor α (TNFα). These activators may be counteracted for instance by antibodies binding to the respective receptor, such as antibodies against the IL-2 receptor.

In a preferred embodiment, the retrodifferentiation inducing agent is selected from the group consisting of
(a) immune inhibitors; and
(b) immune-response modifiers; and
(c) differentiation influencing agents.

"Immune inhibitors" as used herein are agents conferring immune inhibition and/or down regulation of the immune reaction and/or agents that counteract the action of immune activators. In particular, the specific immune reaction is inhibited by inhibitors acting on generation, activity and survival of B- and T-lymphocytes including their products, and acting on generation, activity and survival of antigen-presenting cells, including their products. Alternatively, the unspecific immune reaction is suppressed by agents that inhibit generation, activity and survival of unspecific immune cells such as monocytes, macrophages, granulocytes and their products, and a number of humoral nonspecific components such as complement. Examples for immune inhibitors which may be used in the methods according to the invention are interleukin 10 (IL-10), TGFbeta; interferons; hormones; receptors; immune inhibitory drugs; cytostatics; statins and microbial compounds such as cyclosporin.

Immune inhibitory drugs are prednisolone, dexamethasone, urocanic acid, cyclosporine-A, tacrolimus (FK 506), ursudeoxycholic acid (UDCA), desoxyspergualine (DSG), mycophenolate mofetil (MMF). Similarly, cytostatics act through a general suppression of cell proliferation and by inducing cell death and thereby kill lymphocytes rather than monocytes and thus are also immune inhibitors. Immune-inhibitory cytostatics such as cyclophosphamide, azathioprin and methotrexate are included.

Cytostatics have been designed to kill or to prevent proliferation of tumor cells. In a given concentration, non-tumor cells are kept alive. In a given concentration these drugs are also immune-inhibitory in that they kill proliferating lymphocytes, while keeping alive other cells such as monocytic cells. Actions on surviving cells can be summarized as the side effects of a drug. It appears that the monocyte system is programmed to react, as a side effect, by a genetically pre-programmed reaction pattern in the sense described herein, which can be summarized as the "regenerative program", developing along a pathway which is referred to as "retrodifferentiation" herein. This program appears to be activated generally in all cases, where an "activation" or "forward differentiation" of the monocytes is blocked.

The term "immune response modifier" is used synonymously with "immunomodulating agent" and "immune regulatory agent" and refers to the body's innate regulatory molecules and immunologically active drugs, the function of which cannot clearly be attributed to either immune activation or immune suppression in general. "Immune response modifiers" are agents that have been described to influence the direction of an immune response. The most prominent branching of an immune reaction known to occur within the T-helper cell system. The T-helper cells can either be polarized into the Th1 or into the Th2 direction. Signals to control this branching are mutally excluding each other, meaning that a signal that provokes one direction at the same time is inhibitory for the other direction. For instance, interferon-gamma induces the Th1 polarization and inhibits the Th2 direction. In contrast, IL-4 promotes the Th2 direction and inhibits the Th1 direction. It appears that every immunomodulating agent in parallel activates one and inhibits another developmental direction.

"Immune response modifiers" include thymus peptides (such as Thym-Uvecal, Strathmann), and endogenous mediators such as IL-4.

"Differentiation influencing agents" refer to agents which directly interfere with the process of differentiation. These agents either induce one defined process of "forward" differentiation, or they rather act on the cells to render them reprogrammable in a general sense. Most of them are typically used in combinations or cocktails with other inducers to induce a particular process of differentiation. Because they are not restricted to one particular type of differentiation, they are typically found in recipes of various differentiation protocols.

"Differentiation influencing agents" are heterogenous substances which have been found to induce differentiation in a given test system. The particular direction of differentiation is characterized by the fact that various other directions of possible differentiation are at the same time suppressed by the same substance. It therefore appears that every agent that induces one direction of differentiation at the same time is a suppressor of others. For instance, VEGF is characterized by its double function, namely inducing vascular endothelial growth as a differentiation inducing factor, and in parallel to prevent other differentiation pathways, in this case preferentially to inhibit development of myeloid dendritic cells.

Examples for compounds which may be used as retrodifferentiation inducing agents in the methods according to the invention for producing pluripotent intermediate cells are shown in Table 2.

As a general principle, the heterogenous agents listed in Table 2 all exhibit more than one effect, most of them usually summarized as "side effects". None of these agents is only an activator or inhibitor; rather, they rather inhibit one system while activating another system. These polarities are reflected in columns 3 and 4 of Table 2. With respect to monocytes and the related cells of the "myeloid lineage" of bone marrow cells, it appears that these cells react on those manifold influences by a homogenous, genetically pre-determined program of "retrodifferentiation".

According to Table 2, the following categories of retrodifferentiation inducing agents exist:
- 1: Immune Inhibitors
- 1.1: Interleukins / Immune Mediators
- 1.2: Interleukins / Immune Mediators of the Interleukin-10 Family
- 1.3: Signal transmission: Prostaglandin System
- 1.4: Signal transduction:
- 1.5: cAM P/cG M P-system
- 1.6: Steroids
- 1.7: Interferons
- 1.8: Receptors
- 1.9: Receptor-antagonists
- 1.10: Immune inhibitory drugs
- 1.11: Immune-inhibitory Cytostatics
- 1.12: Statins
- 1.13: Mikrobial /Plant-derived immune inhibitory components
- 2: Immune-Response Modifiers
- 2.1: Interleukins / Immune Mediators
- 2.2: Thymus peptides
- 3: Differentiation Influencing Agents
- 3.1: Vitamins
- 3.2: Neuropeptides
- 3.3: Histone deacetylase (HDA) inhibitors
- 3.4: Angiotensin-System
- 3.5: Extracellular Matrix
- 3.6: Others

**Table 2:**

| Examples for retrodifferentiation inducing agents | | | | |
|---|---|---|---|---|
| | **Synonyms, Abbr., Source, Product Name** | **Mechanism: Inhibition, Antagonism** | **Mechanism: Activation** | **Experi- mental Dosage** |
| **IMMUNE INHIBITORS Interleukins** / **Immune Mediators / Cytokines** | | | | |
| Transforming growth factor beta | TGFbeta | Th1, macrophages | B-lymphocytes: IgA-synthesis, Langerhans cells; osteoblast, fibro-blast | 3.125 ng/ml |
| Vascular endothelial growth factor | VEGF | dendritic cell development | Differentiation inducer for endothelia and blood vessels | 6.25 ng/ml |
| Urocanic acid | UCA | natural product in the skin and liver, cis-urocanic acid formed after UV-irradiation. dendritic cell movement and recruitment | | |

| **Interleukins /immune Mediators of the Interleukin-10 Family** | | | | |
|---|---|---|---|---|
| IL-10 | | inhibitor for T-helper cells 1 (Th1) | Th2, proliferation of B-lymphocytes | 1.562 ng/ml |
| IL-19 | | as IL-10 | as IL-10 | |
| IL-20 | | as IL-10 | as IL-10 | |
| IL-22 | | as IL-10 | as IL-10 | |
| IL-24 | | as IL-10 | as IL-10 | |
| IL-26 | | as IL-10 | as IL-10 | |

| **Signal transmission: Prostaglandin System** | | | | |
|---|---|---|---|---|
| Prostaglandi n-E-2 | PGE-2 | lymphocytes cGMP | macrophages, cAMP | 78 ng/ml |
| Dinoproston | Minprostin | | | 195 pg/ml |
| Cyclopentone | | | | |
| Leukotriene B4 | | | | 312.5 ng/ml |

| **Signal transduction: cAMP/cGMP-system** | | | | |
|---|---|---|---|---|
| dibutyryl cAMP | | intracellular cGMP | increases intracellular cAMP | |
| Forskolin | | intracellular cGMP | Adenylate-cyclase activator | |
| Pentoxifylline | Trental | intracellular cGMP | Phosphodiesterase-Inhibitor | 2.5 µg/ml |
| Theophylline | | intracellular cGMP | Phosphodiesterase-Inhibitor | |
| Aminophyl-line | | intracellular cGMP | Phosphodiesterase-Inhibitor | |
| Motapizone | | intracellular cGMP | Phosphodiesterase-Inhibitor | |
| Rolipram | | intracellular cGMP | Phosphodiesterase-Inhibitor, nerve growth | |
| IBMX | | intracellular cGMP | Phosphodiesterase-Inhibitor | |
| A2-adenosine receptor | | intracellular cGMP | A2-adenosine receptor-stimulated cAMP accumulation | |

| **Steroids** | | | | |
|---|---|---|---|---|
| Hydrocortisone | | antiinflammatory, Osteoclasts, macrophages | Osteoblasts | |
| Dexamethasone | | antiinflammatory, Osteoclasts, macrophages | Osteoblasts | |
| Prednisolone | | antiinflammatory, Osteoblasts, macrophages | Osteoblasts | |

| **Interferons** | | | | |
|---|---|---|---|---|
| IFN beta | Fiblaferon | cell proliferation | MHC expression | 0.7812 IU/ml |
| IFN beta 1b | Betaferon | cell proliferation | MHC expression | 78 pg/ml |
| IFN alpha 2a | Roferon | cell proliferation | MHC expression | 31.25 IU/ml |
| IFN alpha 2b | Intron A | cell proliferation | MHC expression | 0.39 IU/ml |

| **Receptors** | | | | |
|---|---|---|---|---|
| Soluble CD163 | | | | |
| Thrombospondin | Sigma | | | |
| Thrombospondin-R | Integrin-assocciated protein | CD47 ligation. a-CD47 inhibits IL-12; Peptide 4N1K | | |

| **Receptor-antagonists** | | | | |
|---|---|---|---|---|
| Anti-IL-2-receptor | | antibody against IL-2 receptor | | |
| Anti-IL12-receptor | | antibody against IL-12 receptor | | |
| RANK | | Osteoblasts | osteoclasts | |
| PD169316, SB203580 | RANK/L -Inhibitors | Osteoclasts | osteoblast | |

| **Immune inhibitory drugs** | | | | |
|---|---|---|---|---|
| Cyclosporin | Sandimun | inhibits calcineurin, CsA required for inhibition of IL-2 induction in the cells with increased Ras activity | fibroblast differentiation | 1.22 ng/ml |
| Cyclosporin | pure | | | 15.625 ng/ml |
| FK-506 | Tacrolimus Prograf | inhibits calcineurin. thio-redoxin (TRX), FK506 and cyclosporin A (CsA) bound to their receptors, FKBP12 or cyclophilin, inhibit the Ca2+/calmodulin-dependent protein phosphatase, calcineurin | osteoblast differentiation | 15,625 ng/ml |
| 15-Desoyspergualin | DSG | suppression of the monocyte/macrophage action and lymphocyte proliferation. Cell differentiation: Deoxyspergualin inhibits nuclear translocation of activated NF-kappaB through heat shock proteins. Inhibits Agpresentation | from Bacillus laterosporus, binds to HSP | |
| Ursudeoxycholic acid | UDCA | | | |
| Rapamycin | Sigma | Antibioticum, immunsuppressive. | Streptomyces hygroscopicus, target of sirolimus inhibition. | |
| Sirolimus | | | not suppressive for IL-12, in contrast to Dex | |
| Leflunomide | isoxazol derivative A77 1726 active metabolite | inhibits IL-2 driven T-cell proliferation, inhibits src-family tyrosine kinase and pyrimidine-biosynthesis. inhibits monocyte aggregation -cell contact | | 6.25 µM |
| Brefeldin-A | Decumbin, Ascotoxin, Sigma | inhibits cytosolic way of antigen-presentation | | |
| mitoxantrone (Mx) | | | | |
| Brequinar | | | | |
| FTY720 | Novartis | influences chemokine-induced migration | Sphingolipid | |
| *Statins* | | | | |
| *Statins + Antioxidants + cylosporin* | | | | |
| *Statins +angiotensin- R blockers, NAD(P)H oxidase in- hib, NO- aspirin* | | | | |
| *Simvastatin* | | | | |
| *Atorvastatin* | | | | |
| *Atorvastatin* | | | | |
| *Cerivastatin* | | | | |
| *Clopridogrel* | | | | |
| *Fluvastatin* | | | | |
| *Lovastatin* | | | | |
| *Pravastatin* | | | | |
| L-*mevalonate, geranyl- geranylpyro- phosphate* | | | | |
| *Compactin* | | | | |

| **Immune-inhibitory Cytostatics** | | | | |
|---|---|---|---|---|
| Cyclophos-phamide | Endoxan | DNA crosslinking: replication | | 244 ng/ml |
| Azathioprin | Imurek | purine nucleotide synthesis | | 976 pg/ml |
| Methotrexate | MTX Hexal | dihydrofolate reductase inhibitor | | 62.5 ng/ml |
| Mycophenolate mofetil | MMF, Cell-Cept | Antimetabolite, Immun-suppressive. non-competitive and reversible inhibitor of inosine monophosphate dehydrogenase (IMPDH) and of the type II isoform of guanosine nucleotides. | Acts on Dendritic cells Macrophages | 195 ng/ml |
| Mepacrine | | Matrix-Metalloproteinase-Inhibitors, c-Jun > C-Fos. Synoviocyten | | |
| Busulphan, Busulfan | Tetrame-thylen-di(metansulf onat) | | | |

| **Statins** | | | | |
|---|---|---|---|---|
| Statins | | HMG-CoA-reductase | osteoblast differentiation | |
| | | inhibitors, lower Neop-terin expression, antiin-flammatory Suppress MHC II, inhibits NFkB | | |
| Statins + Antioxidants + cylosporin | | inhibit HLA-II upregulation on endothelial cells | | |
| Statins +angiotensin-R blockers, NAD(P)H oxidase inhib, NO-aspirin | | inhibit NO and superoxide | | |
| Simvastatin | Zocor | inhibits NFkB | induces alkaline phos-phatase and osteoblasts in stroma cells, increases endothelial progenitors (EPC) in blood | 12.2 ng/ml |
| Atorvastatin | Lipitor | in vitro, not in vivo immunosuppressive | increases No. of endothelial progenitors (EPC) in human blood | |
| Atorvastatin | Sortis | inhibits Neopterin | | 48.8 ng/ml |
| Cerivastatin | Baycol | inhibits bone resorption, Cer>ATV>Sin>Pra>Lov> Flu, inhibits NFkB: tissue factor expression, Th1 polarization | TH2 polarization, | |
| Clopridogrel | Plavix | in vitro, not in vivo immunosuppressive | | |
| Fluvastatin | Lescol, Cranoc | inhibits NFkB Meralonate antidot | Caspase | 48.8 ng/ml |
| Lovastatin | Mevacor, Altocor Mevinacor | inhib NFkB | | 48.8 ng/ml |
| Pravastatin | Pravachol Pravasin | inhibits NFkB | | 390 ng/ml |
| L-mevalonate, geranyl-geranylpyrophosphate | Statin-antagonist | inhibits CD40/CD40L: immunmodulatory via NOS or Peroxisomes, decreases HLA-DR expression | | |
| Compactin | | | combined with retinoic acid and BMP induces osteoblast differentiation | |

| **Mikrobial / Plant-derived immune inhibitory components** | | | | |
|---|---|---|---|---|
| *Cyclosporin* | | | | |
| *Cyclosporin* | | | | |
| 15- | | | | |
| *Desoysper- gualin* | | | | |
| *Rapamycin* | | | | |
| Mannan | | inhib. IL-12 | complement, macrophage activation | |
| Helicobacter pylori (Hp) VacA | | | vacuolating cytotoxin | |
| Shiitake | | | 1:40 | |

| **IMMUNE-RESPONSE MODIFIERS Interleukins / Immune Mediators** | | | | |
|---|---|---|---|---|
| IL-3 | | | | 25 ng/ml |
| IL-4 | | antiinflammatory and immunsuppressive | immune-response modifying: TH2-inducing | 25-50 U/ml |
| IL13 | | antiinflammatory and immunsuppressive | immune-response modifying: TH2-inducing | |

| **Thymus peptides** | | | | |
|---|---|---|---|---|
| Thymus peptides | Thym-Uvocal, Strathmann | | immune-response modifying | 500 ng/ml |
| Thymostimulin | TP1 | | immune-response modifying | |

| **DIFFERENTIATION INFLUENCING AGENTS Growth factors, differentiation factors** | | | | |
|---|---|---|---|---|
| *Vascular endothelial growth factor* | *VEGF* | | | *6.25 ng*/*ml* |
| *Transforming growth factor beta* | | | | |
| *Prostaglan- din-E-2* | | | | |
| *Dinoproston* | | | | |
| *Cyclopento- ne* | | | | |
| *Leukotriene B4* | | | | |
| *Hydrocorti- sone* | | | | |
| *Dexametha- sone* | | | | |
| *Prednisolone* | | | | |
| *Anti-IL-2- receptor* | | | | |
| *Anti-IL 12- receptor* | | | | |
| *RANK* | | | | |
| *PD169316, SB203580* | | | | |
| *IL-10* | | | | |
| *IL-19* | | | | |
| *IL-20* | | | | |
| *IL-22* | | | | |
| *IL-24* | | | | |
| *IL-26* | | | | |
| *IFN beta* | | | | |
| *IFN beta 1b* | | | | |
| *IFN alpha 2a* | | | | |
| *IFN alpha 2b* | | | | |
| *dibutyryl cAMP* | | | | |
| *Forskolin* | | | | |
| *Pentoxifylline* | | | | |
| *Theophylline* | | | | |
| *Aminophylli- ne* | | | | |
| *Motapizone* | | | | |
| *Rolipram* | | | | |
| *IBMX* | | | | |
| *A2- adenosine receptor* | | | | |
| *Cyclosporin* | | | | |
| *FK-506* | | | | |
| *Mannan* | | | | |

| **Vitamins** | | | | |
|---|---|---|---|---|
| Vitamin A | retinoic acid | differentiation block induced by expression of Scl and Rbtn2 transcription factors | stem cell renoval, neuronal and granulocyte differentiation, skews monocyte differentiation into interleukin-12-secreting dendritic-like cells | 1.562 µM |
| Vitamin C | ascorbic acid | reactive oxygen species scavanger | | |
| Vitamin D | | | osteoblast differentiation | |
| Vitamin E | Alpha-Tocopherol | reactive oxygen species scavanger | spermiogenesis | |
| Vitamin K2 | menaquinone-4 | inhibits Osteoclasts Rifampicin + Hyperforin synergistic with Vit. K | Osteoblast differentiation, alkaline phosphatase | 781 nM |

| **Neuropeptides** | | | | |
|---|---|---|---|---|
| Vasoactive intestinal peptide | VIP | | | |

| **Histone deacetylase (HDA) inhibitors** | | | | |
|---|---|---|---|---|
| Apicidin | | also inhibits Matrix Metal-loproteinases | | |
| Apicidin | | | | |
| Trichostatin-A | | | | |
| HC toxin | | | | |
| ACE inhibitors | | downregulate IL-6 | upregulate IL-10 | |
| Butyric acid | | | | 48.8 µM |
| Buryrate | | | | |

| **Angiotensin-System** | | | | |
|---|---|---|---|---|
| Losartan | Cozaar | AT1-R-Antagonist | | |
| Candesartan | Atacand | AT1-R-Antagonist | | |

| **Extracellular Matrix s.a. Apicidin, Mepacrine** | | | | |
|---|---|---|---|---|
| Pirfenidone | | | | |
| heparan sulfate | | | | |
| chondroitin sulfate | | | | |

| **Others** | | | | |
|---|---|---|---|---|
| Dimethyl sulfoxide | DMSO | | | 0.16%; range 2%-0.000 625% |
| Platelet factor 4 | PF-4 | | | |
| Thalidomide | | angiogenesis inhibitor, TNFalpha synthesis | | |

In the methods according to the invention, two or more retrodifferentiation inducing agents may be used in combination. In a preferred embodiment, the retrodifferentiation inducing agent is selected from interleukin-4 and interleukin-13. IL-4 acts as an inducer of retrodifferentiation, attributed to its character as immune suppressor (regarded by some authors) or as immune response modifier (by its function to trigger a Th2-type of response). Because of their close relationship, IL-4 may be replaced by IL-13. IL-4 may be used either alone or in combination with IL-13.

In another preferred embodiment, dexamethasone sodium phosphate is used as the retrodifferentiation inducing agent. Dexamethasone is an artificial analog of the natural hormone hydrocortisone. Alternatively, hydrocortisone may be used.

According to the present invention, the culture medium used for converting monocytes to pluripotent intermediate cells may optionally further contain granulocyte-macrophage colony stimulating factor (GM-CSF). GM-CSF is regarded as supporting the cell viability and the long-term survival of the cells. As GM-CSF can also be synthesized by the monocytes in an autocrine way, it does not necessarily have to be added to the culture.

In a preferred embodiment, the monocytes used in the methods according to the invention are derived from blood. Preferably, the blood is mammalian blood. Human blood is particularly preferred.

Alternatively, monocytes may be recovered as mature monocytes from bone marrow cells before they immigrate into the blood. Moreover, monocytes may be recovered from their natural precursors, i.e. from bone marrow cells of the "myeloid lineage". Beginning with the haematopoietic stem cell and continuing along the myeloid lineage, monocytes may be produced from any of their precursors using known growth factors such as GM-CSF and M-CSF.

In a preferred embodiment of the present invention, the immune inhibitors and, if present, the GM-CSF are present in physiological concentrations. The term "physiological concentration" as used herein refers to a concentration similar (i.e. deviating by no more than a factor of 5) to that found in blood. In case that natural hormones are used, the natural concentration in blood may easily be measured and compared to the concentration applied in cell culture. For instance, the level of corticosterone in human blood is about 10 microgram per liter blood, corresponding to 3 x 10 ⁻⁸ M; the concentration of cortisol is about 100 microgram/liter, corresponding to 3 x 10⁻⁷ M. During pregnancy, early childhood and certain conditions these levels may differ. As for artificial hormone analogs such as dexamethasone, the cell culture concentration of dexamethasone used according to the invention is about 1 x 10⁻⁶ M, i.e. only 3x higher than the physiological cortison level. A typical dose of dexamethasone if applied therapeutically is 2 mg/liter blood, corresponding to 5 x 10⁻⁶ M. Hence, the concentration of dexamethasone used for retrodifferentiation according to the invention ranges within the physiological normal level of its natural counterpart and is even lower than the concentration used therapeutically.

For cytokines such as IL-4, the concentration cannot be defined as "physiological blood concentration", as cytokines function as short-range factors in the tissue, and are not normally released into the blood. Further, they are usually not therapeutically applied as drugs. Therefore, as far as cytokines such as interleukins (e.g. IL-4) are concerned, "physiological concentration" is to be understood in the sense that their concentration is much lower (i.e. by at least a factor of 10) and therefore more physiological, than the concentrations used in cell cultures previously described in the state of the art. For example, in a previous method for differentiating dendritic cells from monocytes, 300-1000 U/ml IL-4 were applied and GM-CSF was used at 300-500 U/ml. In the methods according to the present invention, the concentrations used for producing the pluripotent intermediate cells in the first step of transdifferentiation are 25 U/ml for IL-4 and 3 U/ml for GM-CSF.

Preferably, the culture medium used in the methods according to the invention is devoid of differentiation promoting factors. The term "differentiation promoting factor" as used herein refers to any compound inducing the differentiation of monocytes and thus hindering the production of pluripotent intermediate cells from monocytes, e.g. factors contained in serum, factors that favor macrophage and/ or adipocyte development, and factors released by (activated) lymphocytes. Factors that hinder the production of pluripotent intermediate cells from monocytes include macrophage colony stimulating factor (M-CSF), fungi, dead or vital microorganisms such as bacteria, lipopolysaccharides, teichoic acids or muramyl peptides and other factors released from microorganisms, granular material, immune complexes, dead cells, and cell fragments. Factors that favor adipocyte development include lipids, liposomes, and chylomicrones. Lymphocytes, especially activated lymphocytes, which release immune activating factors such as interleukin-2 and interferone γ are also inhibitory for the reaction and should therefore be omitted or suppressed.

In order to avoid the presence of differentiation promoting compounds in the method according to the invention, preferably serum-free culture medium is used. Further, the culture medium is preferably depleted of lymphocytes. "Depleted of lymphocytes" means that lymphocytes are basically absent from the culture medium. The lymphocytes may be removed by standard techniques in order to receive an at least 70% pure monocyte preparation, preferably an 80-90% pure monocyte preparation, more preferably a more than 95% or even a more than 99% pure monocyte preparation.

In another aspect, the present invention is related to a method for producing a differentiated cell comprising subjecting the cell population containing pluripotent intermediate cell(s) obtainable according to the method of the invention to differentiation by culturing the cell(s) in the presence of a differentiation inducing agent.

The term "differentiation" refers to a process in which a cell reaches a new state, associated with the acquisition of new properties. Differentiation may be induced by exogenous stimuli. After having reached the new state of differentiation, however, this state is maintained also in the absence of a further stimulus. In contrast, the process of activation leads to a shifting of a resting function to a higher metabolic activity, usually stimulated by exogenous stimuli. When omitting the stimulus, an activated cell falls back to the resting state within hours or maximally 3 days.

The differentiated state must not be a final state. A differentiated cell may further differentiate into another state of differentiation. Differentiation has so far been understood as a "forward"-process, i.e. cells develop away from the embryonic state and away from pluripotency or even totipotency. Recently, it is discussed in the literature that said process of "forward" differentiation may be reversible under physiological conditions. The reversing process is designated as "retrodifferentiation" or "reverse differentiation" and means that cells re-approach the state of pluripotency or even totipotency.

Theoretically, all phenotypes possible may be produced by subjecting the intermediate cell(s) according to the present invention to differentiation by culturing the cell in the presence of appropriate differentiation inducing agents. That is, the intermediate pluripotent cell obtainable according to the methods of the present invention may be differentiated into both hematopoietic cells and non-hematopoietic cells (e.g. osteoblasts, follicular dendritic cells, osteoclasts, hepatocytes, fibroblasts, cartilage cells , endothelial cells, adipocytes, muscle cells, neural cells such as glial and nerve cells).

Specifically, the present invention is related to a method for producing follicular dendritic cells (FDC) from monocytes comprising subjecting the pluripotent cells obtainable according to the methods of the present invention to differentiation by culturing the cell(s) in the presence of tumor necrosis factor alpha (TNFα) and/ or interferone gamma (INFγ). The phenotype of the mature FDCs which may be produced by the method of the present invention may be taken from Table 3. As shown in column six of said table, the monocyte-derived FDCs (MoDC) are positive for alkaline phosphatase, CD35, CD54, and CD106. Moreover, the monocyte-derived FDCs show further characteristics of FDCs, such as rosetting with B-lymphocytes, emperipolesis of B-lymphocytes, giant cell formation, antigen trapping, and expression of B-cell markers.

**Table 3:**

| Markers and functions relevant to osteoblasts, osteoclasts and follicular dendritic cells as compared with classical monocyte-related cells expressed in cultures of monocyte-derived cells. Markers are expressed as % positive cells, functions are given as present (+, ++), absent (-) or diverse (+±) . (T=tested by the inventors) Abbreviations: Macroph. = macrophage; MoDC = monocyte-derived dendritic cell; MoFDC = monocyte-derived follicular dendritic cell; OB = osteoblasts; OC = osteoclasts; d = day of culture; BC = B-cells = B-lymphocytes; TC = T-cells = T-lymphocytes; HSC = haematopoietic stem cells; VNR = vitronectin-receptor; Stem cell F R = stem cell factor receptor; MSC = mesenchymal stem cell; +, ++, +++ = positive at three degrees; - = negative, ± = weakly or variably positive;_T = tested. | | | | | | | | | |
|---|---|---|---|---|---|---|---|---|---|
| Marker | Expression | Monocyte, d 0 | Makro-phage d 15 Serum | Makro-phage d 15 GMCSF | MoDC d 7 GMCSF, IL4 | pluripotent interm. cell (retrodifferentiated monocyte) d15 | MoFDC d 18 | OB d 21 | OC d 10 |
| HLA-DR | | (+)T | +T | +T | ++T | +T | ++T | | |
| CD1a | | | | | + | | | | |
| CD11b (MAC-1) | | + | | | | | | | |
| CD14 | | + | | + | - | | | | |
| CD16 | | | + | + | - | | | | |
| CD19 | | - | | - | - | | + | | |
| CD21 | BC, FDC | - | | - | - | | ±T | | |
| CD22 | BC | - | -T | -T | - | ±T | ±T | | |
| CD34 | HSC | - | | - | - | | | | |
| CD35 (CR1) | Ery, FDC, BC | + | ca. 10% + | ca. 10% + | | ++T | ++T | | |
| CD51/C D61 | Vitronec-tinR VNR osteo-clasts | - | - | - | - | - | ± | - | +++ T |
| CD54 (ICAM-1) | | + | | | | -T | ++T | | |
| CD68 | | + | + | + | + | +±T | +±T | | |
| CD86 | | + | | | + | +±T | | | |
| CD106 (VCAM-1) | Endothelial cells, FDC | | | | | -T | +T | | |
| Alkal. P'ase | | -T | -T | -T | - | +++T | +++T | | |
| Clustering | | +T | - | - | +T | ++T | +++T | | |
| B-cell-rosetting | | - | -T | -T | + | ++T | ++T | | |
| Emperipolesis | | - | -T | -T | - | +T | +T | | |
| Antigen-Trapping | | - | -T | -T | - | +T | +T | | |
| Osteo-calcin | Osteoblasts | - | - | - | - | - | ± | +++ T | - |

The present invention is further related to a method for producing osteoblasts from monocytes, comprising subjecting the pluripotent intermediate cell(s) obtainable according to the methods of the present invention to differentiation by culturing the cell(s) in the presence of at least one bone morphogenetic protein. Bone morphogenetic protein-2 (BMP-2) is preferred. Alternatively or additionally, BMP-3 and/or BMP-4 may be used.

As the primary marker for defining the osteoblast development, the expression of osteocalcin may be used. The phenotype of the osteoblast derived by the method according to the present invention can be taken from Table 3.

Even further, the present invention is related to a method for producing adipocytes from monocytes, comprising subjecting the pluripotent intermediate cell(s) obtainable according to the methods of the present invention to differentiation by culturing the cell(s) in the presence of isobutylmethylxanthin and/or insulin and/or dexamethasone. Preferably, isobutylmethylxanthin, insulin, and dexamethasone are used in combination. The differentiation into adipocytes may be supported by lipids, including cholesterol. To identify adipocytes, adipocytes may be highly specifically stained by the fat stain Oil-redO.
As mentioned above, theoretically, any phenotype may be produced by subjecting the intermediate cell(s) according to the present invention to differentiation by culturing the cell in the presence of appropriate differentiation inducing agents. For instance, the induction of forward differentiation from intermediate pluripotential cells to the following cells types may be achieved under the following conditions:

Osteoclasts are differentiated by adding RANK-L, GM-CSF, M-CSF, interferon gamma. Markers for osteoclasts are known in the art (e.g. tartrate-resistant acid phosphatase, vitronectin-receptor and calcitonin-receptor.) The function of osteoclasts may be tested by plating them on dentin plates or dentin-coated surfaces where they produce defects in the dentin.

Inducers for the production of fibroblasts are fibroblast growth factor (FGF) (10 ng/ml) and/or TGFbeta (1-10 ng/ml). As a specific marker, alpha-1-hydroxylase may be used.

Differentiation into myocytes (muscle cells) may be induced by basic fibroblast growth factor (4ng/ml), and/or 5-azacytidine (10µM), and/or 50 mM hydrocortisone. As a marker, anti-myosin antibody may be used.

Inducers for endothelial cells are VEGF (1-10 ng/ml) and/or hydrocortisone (50 mM). As markers, van Willebrand factor, CD34, CD144, CD105, and/or VEGF-receptor-1 may be used.

Differentiation into hepatocytes may be induced by hepatocyte growth factor (100 ng/ml). For identifying hepatocytes, immunostaining for albumin may be used.

The intermediate cell(s) according to the invention may further be induced to differentiate into nerve cells using embryonic stem cell medium supplemented with 0,1 M beta-2-mercaptoethanol, 1 % non-essential amino acids and nerve-growth factor (200 ng/ml). As a marker, anti-glial fibrillary acidic protein-antibody may be used.

In yet another aspect, the present invention relates to a cell population containing a majority of pluripotent intermediate cells which may be obtained by the method according to the invention, according to which highly purified monocytes are cultured in the presence of at least one retrodifferentiation inducing agent and optionally GM-CSF, until a majority of the monocytes has obtained the state of an intermediate cell, and subsequently recovering the cell population. Such a cell population contains at least 50% of pluripotent intermediate cells and preferably more than 50% of pluripotent intermediate cells. As indicated above, in a preferred embodiment, the percentage of pluripotent intermediate cells is at least 60% or even 70%. In a particularly preferred embodiment, the percentage is more than 80% or 90%. In one embodiment of the invention, the pluripotent cells are characterized in that they have an AP+ phenotype.

The present invention further relates to a pluripotent intermediate cell obtainable by culturing highly purified monocytes in the presence of at least one retrodifferentiation inducing agent and optionally GM-CSF; and subsequently recovering the pluripotent intermediate cells. In one embodiment of the invention, the pluripotent cells are characterized in that they are having an AP+ phenotype. AP positive cells may be harvested by standard cell culture techniques known in the art.

In a preferred embodiment, the pluripotent cell is characterized in that the cell is variable for CD35 and negative for interleukin 1β and TNFα.

In yet another aspect the present invention relates to the use of the pluripotent cell of the present invention obtainable by the methods according to the present invention in substitutional therapy. The term "substitutional therapy" as used herein includes "cell substitution therapy" (adoptive cell therapy) and refers to a therapy comprising the transplantation of a recipient with cells, and/or tissue or tissue-like structures, wherein the transplanted cells and/or tissues complement a defect in the recipient. The pluripotent cells of the invention may be expanded *in vitro* to form tissue-like structures which, after *in vitro* differentiation to e.g. follicular dendritic cells, chondrocytes, endothelial cells or osteoblasts, may be transplanted to a recipient to substitute for tissues such as cartilage, blood vessels or bone, respectively.

The intermediate pluripotent cells according to the invention can be used as somatic stem cells in adoptive cell therapy of several diseases to repair defects and to induce regeneration. The intermediate pluripotent cells have reduced marker expression of the myeloid lineage and can even be negative for e.g. CD14 and CD68. They have also reduced expression of molecules of the major histocompatibility complex (MHC) class I and II. Therefore, it may be anticipated that they are less or not rejected when they are transplanted allo- or xenogenically.

The intermediate pluripotent cell as well as all possible phenotypes derived from it carry the potential to be used for adoptive cell therapy. In an autologous setting, cells may be developed *in vitro* according to the methods described herein and re-transplanted into the donor in order to repair defects. In an allogeneic setting, cells from genetically intact individuals may be useful to repair defects in individuals having genetic disorders. After being administered to a subject, the intermediate pluripotent cells may migrate to the sites of defects and, induced by the local environment, further differentiate into the respective tissue relevant phenotypes. The pluripotent cells of the present invention may thus further be used for preparing a medicament for treating diseases caused by genetic defects. In a preferred embodiment, the genetic defect is cystic fibrosis, sickle cell anemia, hemophilia, Down's syndrome and/or Turner syndrome.

In further aspects, the present invention relates to the use of the follicular dendritic cells (FDCs) obtainable by the method according to the invention for research, diagnostic and therapeutic purposes. The FDC developed from the pluripotent precursors (pluripotent intermediate cells) according to the invention may e.g. be used for immunological research such as studying the persistence of pathogens such as prions and HIV. Specifically, monocyte-derived FDCs may help to elucidate functioning of the immunological memory. The extent of antigen trapping relevant for pathogenic organisms such as HIV may be investigated, as it is still open whether the virus can exist within FDCs in an infectious form or even may replicate within FDCs.

In another aspect, the follicular dendritic cells obtainable by the methods according to the invention may be used for diagnostic purposes. For instance, FDCs from an uninfected donor may be incubated with blood or serum sample(s) of an individual to be tested. If present, infectious agents will be collected or even be amplified within the previously uninfected FDCs and may subsequently be identified by methods known in the art, such as agar culture or polymerase chain reaction. Further, different isolates of infectious agents may be added to separate cultures of FDCs and their trapping within the FDCs may be determined in order to compare their infectivity and their persistency.

Moreover, in another aspect, the follicular dendritic cells obtainable by the method according to the invention may be used for therapeutic purposes other than gene therapy. In a preferred embodiment, the follicular dendritic cells are used in substitutional therapy.
The FDCs obtained by the method according to the present invention may further be used for preparing a medicament for treated infectious diseases. In a preferred embodiment, the infectious disease is caused by HIV.

The FDC is the last of the major immune cells who's origin was unclear so far. Facing the enormous impact of FDCs on the function of the immune memory, and their function as natural reservoir of pathogens (HIV, prions), the discovery of their ontogenesis is of high value because as a consequence, FDCs may be produced from their precursors in cell culture, their development may be influenced *in vivo* and thus immune reactions and vaccination strategies may be modified and improved. Regarding their value in infection research, using the FDCs according to the invention, it will be testable whether pathogens such as viruses (e.g. HIV), bacteria, protozoa and prions (e.g. BSE) are resident within these cells with a chance to remain pathogenic for a long time. In such a test, a culture of FDCs obtained from a non-infected donor may for instance be treated with HIV-virus. As a control, monocytes or macrophages from the same donor may be used. It will be tested how long the FDCs can entrap the infectious agent by harvesting material from the culture and testing it in an independent infection test, e.g. using polymerase chain reaction. Similarly, any other pathogen may be tested as well. The result provides information as to how far these pathogens use this cell to be protected against destruction. As known from isolated tonsillar FDCs, the antigen is trapped by enrolling into membrane pockets and thereby protected against exogenous as well as cellular lytic events ( Habermannand Shlomchik, 2003).

FDCs generated by the method according to the invention are particularly useful for immunological research and for cell transplantation whether in an allogeneic, autologous or xenogeneic setting. By use of the FDCs according to the invention, immunological diseases may be influenced. In particular, FDCs obtained according to the methods of the invention may be charged with drugs, rendering them resistant against a given pathogen and subsequently may be injected in an individual suffering from an infectious disease to compensate for infected FDCs corrupted by a surviving pathogen.

In other aspects, the present invention relates to the use of osteoblasts obtainable by the methods according to the invention for research purposes, diagnostic purposes and therapeutic purposes. The possibility to generate osteoblasts from monocytes is a recent finding, inasmuch as osteoblasts have so far been regarded to develop from mesenchymal stem cells or stromal cell within the bone marrow. The osteoblasts obtained by the method according to the invention may be utilized to investigate the balance of regulation between osteoblasts and osteoclasts. In particular, the signals which mediate the development into either direction may be tested. Regarding diagnosis, using the osteoblasts according to the invention, from patients suffering from bone disorders such as osteoporosis may be tested whether their monocytes already carry the preference in favour of osteoclastic reaction. It can be tested if there are signals in the patient's serum which induce an osteoclastic reaction.

In a preferred embodiment, the osteoblast obtained by the method according to the present invention are used in substitutional therapy. For this purpose, monocytes from a patient may for instance be differentiated into osteoblasts *in vitro,* harvested by conventional cell culture techniques (as described), washed free of cell culture medium and of the differentiation inducing agent(s) and then injected into the recipient either systemically (e.g. introvenously) or locally at the site of a defect.

Moreover, the osteoblasts obtainable by the method according to the invention may be used for preparing a medicament for treating osteoporosis and bone defects.

Since retrodifferentiation inducing agents are able to induce retrodifferentiation from monocytes to intermediate pluripotent (AP+) cells, this process may be used to test unknown substances for their retrodifferentiation-inducing potency. For instance, pharmaceuticals in development may be subjected to this new test system. The read out is performed by counting the percent of AP+ cells. Alternatively, an AP reaction may be performed according to standard procedures resulting in a soluble end product which can then be quantitatively measured by an ELISA-reader. In addition to searching for pharmaceuticals, the immune-suppressed state of a patient may be measured by adding his serum to the monocyte culture. This applies for the blood of patients who have received an immunosuppressive therapy as well as to patients who have developed immunosuppression caused by a disease, such as tumors, which are known to exhibit strong immunosuppressive activities. In both cases, the measurement of drugs as well as the measurement of endogenously developed immune suppression the test is able to cover the effect of several activities collectively.

In yet another aspect, the present invention relates to a method for identifying new retrodifferentiation inducing agents comprising the steps of:
- culturing monocytes obtained from healthy blood donors,
- adding the new agent or factor to be tested to the culture, and
- after 3-28 days of incubation, testing the cells for their ability to transdifferentiate into another cell type,
wherein the agent is considered to induce retrodifferentiation if at least 50 percent of the cells are capable of transdifferentiation.

Preferably, 70% of the cells are capable of transdifferentiation, more preferably over 90%, most preferably over 95% of the cells are capable of transdifferentiation.

Applying the method according to the invention, agents inducing retrodifferentiation of monocytes such as chemical drugs, natural plant and animal products, human products such as cytokines and growth factors may be newly identified.

### FIGURES

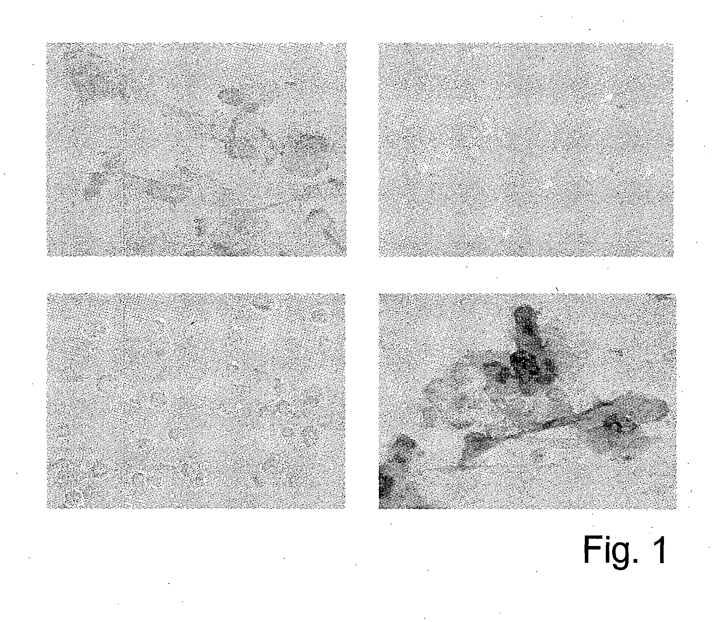

Fig. 1. Expression of phenotypes developed from human monocytes. Monocytes were treated either with IL-4 alone (upper left), with GM-CSF alone (lower left), with dexamethasone alone (upper right) and with IL-4, GM-CSF, and dexamethasone (lower right). Blue: alkaline-phosphatase.

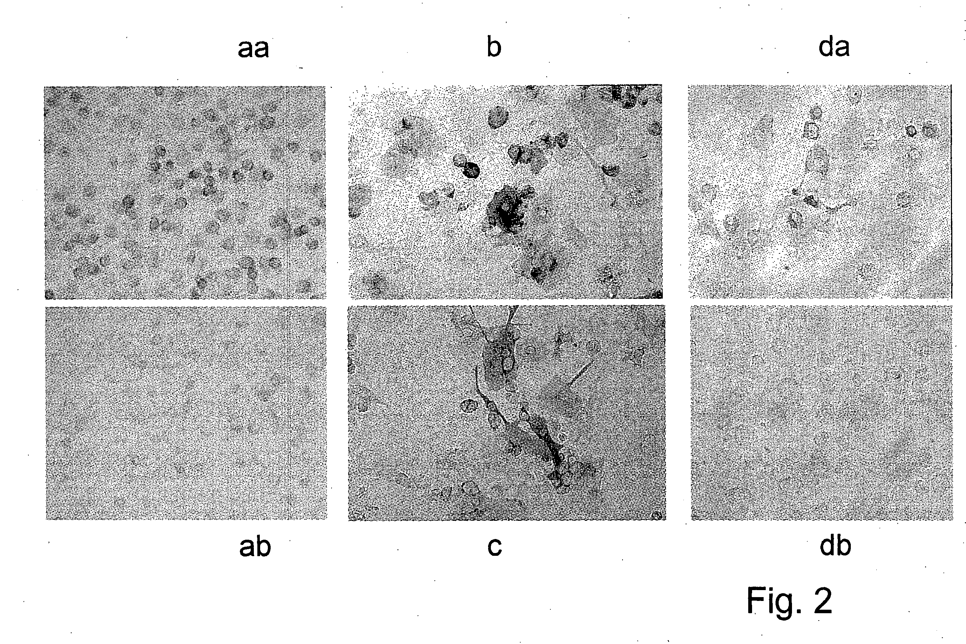

Fig. 2. Monocyte-Derived Follicular Dendritic Cells (FDC) (aa) MoFDC day 15, stained for CD35 expression (brown); (ab) Macrophage control, stained for CD35 expression; (b) MoFDC day 14 stained for AP (blue), rosetted with B-lymphocytes, cell line Raji, stained for CD22 (brown). (c) MoFDC day 14, emperipolesis (uptake) of B-lymphocytes (Raji). AP (blue), rosetted and emperipolesed B-lymphocytes, cell line Raji, stained for CD22 (brown). (d) Antigen Trapping. FDC obtained from monocytes after 12 days of stimulating with dexamethasone, IL-4 and GM-CSF, received immune complexes with horse radish peroxidase. After another 4 days the cultures were fixed and reacted with diamino benzidine and H₂O₂ for a peroxidase reaction (brown) FDC were shown to stain positive (da). whereas control macrophages were >99.9% negative (db).

Fig. 3. Monocyte-derived Osteoblasts. Osteocalcin demonstrated by immunocytochemical reaction against osteocalcin in the nuclei (red). AP (blue), CD86 (brown).

### EXAMPLES

### Introduction

The present authors have previously observed alkaline phosphatase (AP) to be unexpectedly expressed in monocyte cultures either obtained from ex-vivo or in-vitro induced granuloma, suggesting osteoblastic differentiation. AP of the "osteoblast type" has furthermore been shown to be expressed in osteoblasts, activated endothelial cells as well as in follicular dendritic cells (FDCs; Rademakers). The present authors therefore hypothesized that AP expression may serve as a common link for differentiating those cells from monocytes and attempted to find the conditions to provoke AP expression quantitatively in monocytic cells.

Testing this hypothesis, it appeared obligatory to start with highly defined monocyte populations. In order to obtain highly purified monocyte populations, selective adherence of monocytes, combined with the method of reverse sedimentation was used. This results in a very thorough depletion of lymphocytes, leaving monocytes untouched by antibodies or magnetic particles. Autologous as well as pooled human or fetal calf sera yielded positive results. Serum-free media are preferred to prevent charge variations, leading to highly reproducible production of FDC in various serum-free media.

Dexamethasone combined with IL-4 and GM-CSF prevents monocytes to develop into macrophages or DC. Instead, within two weeks, they developed into a novel cell type, the pluripotent intermediate cell which is phenotypically closely related to FDCs. Starting from this intermediate, an even more mature phenotype of FDCs as well as osteoblasts and adipocytes can be differentiated using appropriate physiological stimuli.

### Preparation of purified monocytes

Monocytes were obtained by leukapheresis from healthy blood donors. Mononuclear cells were prepared by standard Ficoll-Hypaque gradient sedimentation (density 1.077p, Lymphoprep, Nyegard, Oslo, Norway). Alternatively, the gradient was used at 1.068p.

The monocyte content was measured from unstained cells by forward/scatter analysis using a flow cytometer. Mononuclear cells were seeded into microwells at 30 000 monocytes/well, centrifuged at 500g for 1 min. to enhance sedimentation, and allowed to attach for a one hour incubation time. For separating non-attached cells from the monocytes, wells were filled up with culture medium to form a convex meniscus, the microplates were then gently tilted top-down and cultured 2-18 hours in the inverted position. After reverse sedimentation was completed, the medium containing non-attached cells was snicked off, the cells washed once with PBS, and the adherent cells were further cultured in 100 µl of differentiation medium.

### Induction of retrodifferentiation

CellGro serum-free medium (CellGenix, Freiburg, Germany) was supplemented with N-acetyl-L-alanyl-L-glutamin (Ac-ala-gln) (Biochrom, Berlin, Germany), Penicillin-Streptomycin (PAN Biotech, Aldenbach, Germany). Alternatively, serum-containing RPMI 1640 medium was used. Inducers of retrodifferentiation were Dexamethasone, IL-4, and GM-CSF. Dexamethasone was used at 10⁻⁸ - 10⁻⁵M, preferably at 5x10⁻⁷M - 1x10⁻⁶M. IL-4 was used at 5-100 U/ml, preferably at 25-50 U/ml. GM-CSF was used at 1-20 U/ml, preferably at 1-5 U/ml.

Once a week, medium plus additives were completely exchanged. At day 15, the cultures were terminated and tested for their phenotype and function as retrodifferentiated monocytes (pluripotent intermediate cells), or, alternatively, additional inducers were added at day 13 in order to induce "forward differentiation" and the cultures were terminated after additional 3 days. Medium was removed, the cells were fixed with ethanol/acetic acid (95%/5%) and stained. Alkaline phosphatase was stained using a kit from Sigma (Deideshofen, Germany) according to the manufacturer's instructions.

### Harvesting of pluripotent intermediate cells

At the end of the described differentiation period, the cells are harvested according to standard cell culture techniques: they are deprived of calcium-ions by incubating them in phosphate buffered saline lacking Ca++ and Mg++, containing further 0.02 % EDTA, a calcium chelating agent. In order to improve the detachement of the cells, they are kept in the cold (in order to depolymerize microtubuli). Furthermore, they may be treated with 0.01 % xylocaine (Lidocain), a local anaestetic, which has been shown to induce detachment of cells. Alternatively, enzymes such as trypsin (0.25% or trypsin 0.05% w/v in ED-TA 0.02%), collagenase (0.1-0.2% w/v) / dispase (0.6-2.4 U/ml) may be used at standard conditions. After the cells have been detached (as controlled by phase contrast microscopy), they are collected in a centrifuge tube, centrifuged down at low speed (350xg, 5 min), the buffer removed, and the pelleted cells resuspended in a sterile buffer or medium, depending on the further use.

### Monocytes obtained from a proliferating culture

Contrasting to this, monocytes derived from a proliferating culture were used. Monocytes were put into culture as describe above. For inducing monocyte proliferation, GM-CSF was added at 25 U/ml. During the following 14 days the proliferating cells detached from the surface and were washed. Next, they were transferred into new wells. After omitting the proliferation-inducing concentration of GM-CSF, the cells were subjected to the above described "retrodifferentiation"-protocol in order to stop proliferation and to start retrodifferentiation.

### Induction of "forward-differentiation"

### (a) Differentiation into FDCs

For obtaining fully developed FDCs, the AP+ intermediate cells were subjected to tumornecrosis factor alpha (TNFalpha) at 0.1-40U/ml, preferably 5-20U/ml and/or interferon-gamma at 1-300 U/ml, preferably 5-100 U/ml and kept in culture for 1-5 days. They were then fixed as above and stained for FDC markers. Alternatively, they remained in culture and were tested for their function.

Using TNF-alpha or interferon-gamma separately or combined, cells resembling follicular dendritic cells were obtained. As these cells are not unequivocally characterised in the literature, FDCs were identified using a combination of markers and functions (Table 3). Characteristic functions of FDC that can be tested *in vitro* are: spontaneous homogeneous clustering, rosetting with B-lymphocytes, and emperipolesis, i.e. engulfing of B-cells which remain inside the FDC for longer time.

Raji cells (a permanent human B-lymphocyte line) were added to test for rosette formation. After 4 hours the cultures were stopped, fixed and stained for relevant markers. The same set-up was used to test for emperipolesis, which follows the rosetting and can be observed 4-10 hours later. Again, the cells were fixed and stained for relevant markers. In order to test for antigen trapping, the horse raddish peroxidase (HRPO) as HRPO/human IgG immune complexes, containing HRPO-conjugated mouse-anti human Ig, compexed with aggregated human Ig and human serum (0.1 µg/ml final concentration of HRPO) was added, washed out after 15 min and the cells were further cultured for up to 16 days. At the time points indicated they were fixed and tested for enzyme activity using peroxidase staining at standard conditions (substrate diamino benzidine (DAB) + H₂O₂). Fig. 2b shows monocyte-derived cells cultured for 14 days to rosette with Raji cells as well as engulfed Raji cells (Fig. 2c).

Antigen trapping is a most intriguing function of FDC. We show that peroxidase as a reference antigen is taken up by monocyte-derived FDC (MoFDC) and stays enzymatically active for 16 days (Fig. 2 da). For control, macrophages degraded the enzyme within 4 hours (Table 3 and Fig. 2db).

The data demonstrate that FDC differentiation can be induced from peripheral blood monocytes in a quantitative manner.

### (b) Differentiation into osteoblasts

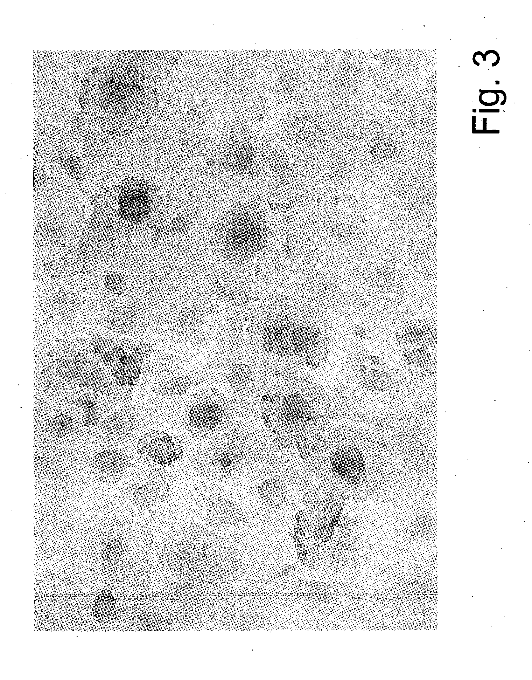

In order to induce osteoblastic differentiation, the AP+ intermediates were subjected to bone morphogenetic protein 2 (BMP-2) at 10ng/ml, 10-1000 nM (preferably 100 nM) dexamethasone, 1-100 mM (preferably 10 mM) beta-glycerophosphate and 1-250 µg/ml (preferably 50 µg/ml) ascorbic acid for 14 days. They were fixed as above and stained for expression of osteocalcin according to standard procedures. Fig. 3 demonstrates the positive reaction for osteocalcin.

### (c) Differentiation into adipocytes

For inducing adipocyte differentiation, AP+ intermediates were further cultured in human serum rich of lipids, or alternatively in medium containing 0.1-50mM (preferably 0.5 mM) isobutylmethylxanthin, 0.1-10 µg/ml insulin (preferably 1 µg/ml) and 0.025-2.5µM dexamethasone (preferably 0.25 µM) for another 3-10 days to induce differentiation of adipocytes. Subsequently, they were fixed and specifically stained with Red Oil O.

### Discussion

### Cell culture for retrodifferentiation

The above results could only be obtained after rigorously improving the cell culture conditions over conventional monocyte culturing. In brief, inhibitory factors deriving from contaminating lymphocytes as well as from certain lots of serum had to be minimized. Human monocytes cultured for 15 days in a combination of IL-4, dexamethasone and GM-CSF (as specified) were flat and adjacent, showing a multiform shape and multiple elongations. A vast majority of the cells expressed alkaline phosphatase. Double staining with CD68 showed the cells to be myeloid in origin. However, upon prolonged culture, expression of CD68 ceased, ending in considerable percentages of cells positive for AP and weakly positive or even negative for CD68. Using the given stimuli, monocytes did not proliferate (shown by the absence of incorporated bromo deoxy uridine, not demonstrated) but rather transformed in a 1:1 fashion into (AP+) pluripotent intermediate cells.

### Forward differentiation

Further stimuli were added to the cells in a subsequent step (see "forward" differentiation above).

### FDC development

Investigating the route of FDC development proved to be complicated, due to the inconsistent marker characterization available for FDCs. FDCs are not characterized by exclusive markers and not uniformly described in the literature. The authors decided to use expression of alkaline phosphatase as an arbitrary marker for FDCs which is negative in monocytes, macrophages and monocyte-derived dendritic cells. As previously described by the present authors, AP expression can be induced in monocytic cells under various conditions such as *in vitro* induced granuloma, and AP expression was detected in ex-vivo cultured foreign body granulomas, suggesting AP expression as an indicator of monocytes' developmental plasticity (Heinemann et al.2000). Therefore, as a first goal we have attempted to convert monocytes into (AP+) pluripotent intermediate cells.

IL-4 was found to be inductive for AP expression in large adherent cells which were flat with fine dendritic protrusions and co-stained for the monocyte marker CD68. This morphology as well as AP expression were further enhanced and stabilized by addition of dexamethasone which is known to stabilize the phenotype of osteoblast cultures and was initially used by us in order to inhibit macrophage differentiation.

After 14 days of culture about 80 % of the monocyte-derived cells displayed this phenotype independent of proliferation. Therefore, the results indicate that the high number of AP-positive cells do not derive from a small subset of clonally expanded AP positive precursors but rather reflect real monocytic differentiation.

Occasionally, these differentiations may spontaneously occur in single cells of conventional cultures as well, such as antigen trapping in a few cells of a macrophage culture and in monocyte-derived dendritic cells (differentiated in a common protocol using GM-CSF plus IL-4 at one order of magnitude higher concentrations than those used above). These findings suggest that monocytes, macrophages and dendritic cells (whether myeloid or follicular) belong to a continuum of interconvertable cells, all of them equipped with the property of being developmentally plastic. The pluripotent nature of these cells is demonstrated by the fact that osteoclasts and osteoblasts, can be as well differentiated from the AP positive intermediate described here.

Therefore, the present authors have found a general route to prevent monocytes to undergo the well-known ("forward-")differentiation toward macrophages or dendritic cells but rather describe a common pathway that allows monocytes to quantitatively convert into an (AP+) intermediate pluripotent cells ("reverse differentiation") which then gives rise to a number of haematopoietic and non-haematopoietic cells.

### REFERENCES

Abe, R., S. C. Donnelly, T. Peng, R. Bucala, and C. N. Metz, 2001, Peripheral blood fibrocytes: differentiation pathway and migration to wound sites: J Immunol, v. 166, p. 7556-62.

Abuljadayel, I. S., 2003, Induction of stem cell-like plasticity in mononuclear cells derived from unmobilised adult human peripheral blood: Curr Med Res Opin, v. 19, p. 355-75.

Fernandez Pujol, B., F. C. Lucibello, U. M. Gehling, K. Lindemann, N. Weidner, M. L. Zuzarte, J. Adamkiewicz, H. P. Elsasser, R. Muller, and K. Havemann, 2000, Endothelial-like cells derived from human CD14 positive monocytes: Differentiation, v. 65, p. 287-300.

Haberman A.M., M.J. Shlomchik, 2003, Reassessing the function of immune-complex retention by follicular dendritic cells. Nat. Rev. Immunol. 3/9: 757-64

Heinemann, D. E., H. Siggelkow, L. M. Ponce, V. Viereck, K. G. Wiese, J. H. Peters, 2000, Alkaline phosphatase expression during monocyte differentiation. Overlapping markers as a link between monocytic cells, dendritic cells, osteoclasts and osteoblasts Immunobiology 202, 68-81

Huss, R., C. Lange, E. M. Weissinger, H. J. Kolb, and K. Thalmeier, 2000, Evidence of peripheral blood-derived, plastic-adherent CD34(-/low) hematopoietic stem cell clones with mesenchymal stem cell characteristics: Stem Cells, v. 18, p. 252-60.

Kuwana, M., Y. Okazaki, H. Kodama, K. Izumi, H. Yasuoka, Y. Ogawa, Y. Kawakami, and Y. Ikeda, 2003, Human circulating CD14+ monocytes as a source of progenitors that exhibit mesenchymal cell differentiation: J Leukoc Biol, v. 74, p. 833-45.

Peters, J. H., S. Ruhl, and D. Friedrichs, 1987, Veiled accessory cells deduced from monocytes: Immunobiology, v. 176, p. 154-66.

Rademakers , L. H. De Weger, R. A., Roholl, P. J. 1988, Identification of alkaline phosphatase positive cells in human germinal centres as follicular dendritic cells. Adv Exp Med Bio 237, 165-69

Rober, R. A., R. K. Gieseler, J. H. Peters, K. Weber, and M. Osborn, 1990, Induction of nuclear lamins A/C in macrophages in in vitro cultures of rat bone marrow precursor cells and human blood monocytes, and in macrophages elicited in vivo by thioglycollate stimulation: Exp Cell Res, v. 190, p. 185-94.

Yang, D., A. Biragyn, L. W. Kwak, and J. J. Oppenheim, 2002, Mammalian defensins in immunity: more than just microbicidal: Trends Immunol, v. 23, p. 291-6.

Zhao, Y., D. Glesne, and E. Huberman, 2003, A human peripheral blood monocyte-derived subset acts as pluripotent stem cells: Proc Natl Acad Sci U S A, v. 100, p. 2426-31.

## Claims

1. Method for producing a cell population containing a majority of pluripotent intermediate cells comprising the steps of
a. culturing a highly purified preparation of monocytes in the presence of at least one retrodifferentiation inducing agent and optionally GM-CSF, until a majority of the monocytes has attained the state of an intermediate cell; and
b. recovering the cell population containing a majority of pluripotent intermediate cells.

2. Method for producing a pluripotent intermediate cell comprising the steps of
a. culturing a highly purified preparation of monocytes in the presence of at least one retrodifferentiation inducing agent and optionally GM-CSF; and
b. recovering a pluripotent intermediate cell.

3. The method according to claim 1 or 2, wherein the pluripotent intermediate cell(s) of step (b) has an alkaline phosphatase positive (AP+) phenotype.

4. The method according to any of claims 1-3, wherein the retrodifferentiation inducing agent is selected from the group consisting of
(a) immune inhibitors; and
(b) immune-response modifiers; and
(c) differentiation influencing agents.

5. The method according to any of claims 1-4, wherein the retrodifferentiation inducing agent is selected from interleukin-4 and interleukin-13.

6. The method according to any of claims 1-4, wherein the the retrodifferentiation inducing agent is dexamethasone sodium phosphate.

7. The method according to any of claims 1-6, wherein the monocytes are derived from blood.

8. The method according to claim 7, wherein the blood is mammalian blood.

9. The method according to claim 8, wherein the blood is human blood.

10. The method according to any of claims 4-9, wherein the immune inhibitor and, if present, GM-CSF are present in physiological concentrations.

11. The method according to any of claims 1-10, wherein the culture medium is devoid of differentiation promoting factors, or immune activating factors which induce differentiation into another direction

12. The method according to claim 11, wherein the culture medium is serum-free.

13. The method according to claim 11, wherein the culture medium is depleted of lymphocytes.

14. Method for producing a differentiated cell comprising subjecting the cell population obtainable according to any of claims 1 or 3-13 or the pluripotent cell obtainable according to any of claims 2-13 to differentiation by culturing the cell(s) in the presence of differentiation inducing agent(s).

15. The method according to claim 14, wherein the differentiated cell is a follicular dendritic cell and the differentiation inducing agent is TNFα and/or INFγ.

16. The method according to claim 14, wherein the differentiated cell is an osteoblast and the differentiation inducing agent is at least one bone morphogenetic protein.

17. The method according to claim 14, wherein the differentiated cell is an adipocyte and the differentiation inducing agent is isobutylmethylxanthin and/or insulin and/or dexamethasone.

18. The method according to claim 14, wherein the differentiated cell is selected from the group consisting of osteoclasts, neural cells, hepatocytes, muscle cells, endothelial cells, and fibrocytes.

19. Cell population containing a majority of pluripotent intermediate cells obtainable by the method of any of claims 1 or 3-13.

20. Pluripotent intermediate cell obtainable by the method of any of claims 2-13.

21. The pluripotent cell(s) according to claim 19 or 20 **characterised in that** the cells do not express CD54, CD106, interleukin 1-beta and TNFα.

22. Use of the pluripotent cell according to any of claims 19-21 in substitutional therapy.

23. Use of the pluripotent cell according to any of claims 19-21 for preparing a medicament for treating genetic defects.

24. The use according to claim 23, wherein the genetic defect is cystic fibrosis, sickle cell anaemia, Down's syndrome, hemophilia and/or Turner syndrome.

25. Use of the follicular dendritic cell obtainable by the method of claim 15 for research purposes.

26. Use of the follicular dendritic cell obtainable by the method of claim 15 for diagnostic purposes.

27. Use of the follicular dendritic cell obtainable by the method of claim 15 for therapeutic purposes.

28. The use of the follicular dendritic cell according to claim 27 in substitutional therapy.

29. Use of the follicular dendritic cell obtainable by the method of claim 15 for preparing a medicament for treating infectious diseases.

30. The use according to claim 29, wherein the infectious disease is caused by HIV.

31. Use of the osteoblast obtainable by the method of claim 16 for research purposes.

32. Use of the osteoblast obtainable by the method of claim 16 for diagnostic purposes.

33. Use of the osteoblast obtainable by the method of claim 16 for therapeutic purposes.

34. The use of the osteoblast according to claim 33 in substitutional therapy.

35. Use of the osteoblast obtainable by the method of claim 16 for preparing a medicament for treating osteoporosis and bone defects.

36. Method for identifying new retrodifferentiation inducing agents comprising the steps of:
- culturing purified monocytes obtained from healthy blood donors,
- adding a new agent or factor to be tested to the culture, and
- after 3-28 days of incubation, testing the cells for their ability to transdifferentiate into another cell type,
wherein the agent is considered to induce retrodifferentiation if at least 50 percent of the cells are capable of transdifferentiation.
